# EUROPEAN PATENT APPLICATION

(11) **EP 4 374 801 A1**
(43) Date of publication of application: **29.05.2024**
(21) Application number: 22209124.1
(22) Date of filing: 23.11.2022
(51) Int. Cl.: A61B 17/22, A61B 17/3207

(54) **LESION CROSSING HELIX**

(71) Applicant: Koninklijke Philips N.V., 5656 AG Eindhoven (NL)
(72) Inventor: MOGGE, James Robert, 5656AG Eindhoven (NL)
(74) Representative: Philips Intellectual Property & Standards

(57) **Abstract**

An intravascular lesion crossing device (10) includes a guidewire support catheter (12) having a guidewire lumen (14) configured to receive an associated guidewire (16) for delivery of a distal end (18) of the guidewire support catheter to an occlusion in a blood vessel; and a helical profile (20) provided at an outer surface of the distal portion of the guidewire support catheter.

## Description

### FIELD

The following relates generally to the catheter arts, catheter guidewire arts, vascular therapy, lesion treatment arts, and related arts.

### BACKGROUND

In catheter-based vascular therapy, a catheter bears one or more tools at its distal end, such as an angioplasty balloon, a laser aperture or cutting tool for thrombectomy or atherectomy, a stent and associated stent deployment hardware, and/or so forth. Initially, a guidewire is inserted into a blood vessel and is fed out until the guidewire crosses past a treatment area (for example, a clot, thrombus, aneurysm, or so forth). The catheter has a guidewire lumen and is inserted along the guidewire into the blood vessel to move the catheter tip to the treatment area. However, total (or near total) occlusions within vasculature (e.g., arteries or veins) are very difficult to cross. This may cause the operator (e.g., physician or surgeon) to poke outside of a main lumen of the vessel to get around the occlusion. If the occlusion is able to be crossed, it generally takes an extremely long time in order for a guidewire to slowly push through the blockage. Additionally, the occlusions comprise a stronger material than the vessel wall, meaning that it is easy to cause a rupture in the vessel while attempting to cross the occlusion, which would require additional intervention to fix. Moreover, in some cases, a physician is completely unable to cross the occlusion, and catheter-based procedure is then aborted.

Guidewire support catheters are commonplace medical devices Guidewire support catheters commonly are low profile devices with a tapered tip whose purpose is to aid medical professionals in delivering a guidewire to and across a treatment site in the vascular anatomy. These devices commonly have an inner diameter close in size to the guidewire size they are intended to support, for example, 0.236 mm, 0.457 mm, 0.889 mm (0.014," 0.018," or 0.035"). Guidewire support catheters have various means to improve their push-ability and torque-ability. There are many options including a shaft of varying inner/outer diameter, a shaft containing braided stainless steel, and a shaft containing stainless steel coils. The achieved push-ability and torque-ability aids these guidewire support catheters when delivering guidewires through various disease states, partial or total occlusions, lesions, and tortuous anatomy.

Crossing of intravascular lesions is often the most time-consuming portion of an intravascular procedure. This can typically take 30-90 minutes to execute, even in experienced hands. In inexperienced hands, it often leads to procedure failure. Moreover, despite advances in guidewire support catheters and techniques, medical professionals still occasionally have difficulty crossing some particularly difficult disease states and tortuous anatomy.

The following discloses certain improvements to overcome these problems and others.

### SUMMARY

In some embodiments disclosed herein, an intravascular lesion crossing device includes a guidewire support catheter having a guidewire lumen configured to receive an associated guidewire for delivery of a distal end of the guidewire support catheter to an occlusion in a blood vessel. In some embodiments a helical profile is provided at an outer surface of the distal portion of the guidewire support catheter. In some embodiments a helical threading is disposed at the distal end of the guidewire support catheter and on an outside of the distal end.

The guidewire support catheter may include a cylindrical core; an inner jacket disposed on an inside surface of the cylindrical core; and an outer jacket surrounding the cylindrical core. The helical threading may be disposed on an outside of the outer jacket or between the cylindrical core and the outer jacket.

In some embodiments disclosed herein, a method of forming a catheter includes coating a core layer with an inner layer; coating a core layer with an outer layer, e.g., heat shrinking an outer layer to the inner layer; and attaching a helical threading to the outer layer.

One advantage resides in providing a guidewire insertion device and corresponding guidewire insertion method providing efficient and safe guidewire crossing of a vascular obstruction or lesion.

Another advantage resides in providing such a guidewire insertion device with a threaded helix to increase an ease of pushing of the insertion device through a lesion.

Another advantage resides in providing a guidewire insertion device with a tapered tip to allow the insertion device to cross a lesion.

Another advantage resides in reducing an amount of time needed for a guidewire insertion device to cross a lesion or obstruction.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURE 1 diagrammatically illustrates a crossing device in accordance with the present disclosure.
FIGURE 2A shows an isolation view of a guidewire support catheter suitably used in the crossing device FIGURE 1.
FIGURE 2B shows an enlarged view of the distal end of the guidewire support catheter of FIGURE 2A.
FIGURE 3 shows a side sectional view of a portion of a distal end of a guidewire support catheter suitably used in the crossing device of FIGURE 1.
FIGURES 4A, 4B, and 4C diagrammatically illustrate three embodiments of the distal end of a guidewire support catheter suitably used in the crossing device FIGURE 1.
FIGURE 5 diagrammatically illustrates a method of forming a guidewire support catheter.

### DETAILED DESCRIPTION

In intravascular treatment of a blood clot, a common workflow is to insert a guidewire up to and past the clot, and then to run a catheter with an interventional tool, such as a laser ablation tool, debulking tool, angioplasty balloon, or the like, to the clot using the guidewire which fits into a guidewire lumen of the catheter. A problem can arise, however, in that the relatively flexible guidewire may be unable to pass through (i.e. "cross") the clot. To address this, a guidewire support catheter can be delivered via the guidewire to the clot. The guidewire support catheter has a small inner diameter (ID) that is just slightly larger than the outer diameter (OD) of the guidewire, but has a stiffer end compared with the guidewire and can therefore be used to penetrate and cross the clot, after which the guidewire can cross the clot through the opening created by the guidewire support catheter.

Crossing the clot using the guidewire support catheter is still challenging. The following discloses a solution in which the tip of the guidewire support catheter (e.g., the distalmost 3-6 cm) has a helical threading is disposed on its OD. The guidewire support catheter can be manually rotated about the guidewire disposed within its guidewire lumen, thereby rotating the tip of the guidewire support catheter and screwing its threaded end into the clot.

The guidewire support catheter typically employs a cylindrical braided or helical stainless steel core designed to provide sufficient flexibility to follow the guidewire to the clot but sufficient stiffness to engage and cross the clot. A smooth inner jacket of a material such as polytetrafluoroethylene (PTFE) or Teflon^{™} coats the inside surface of the cylindrical core, and a smooth outer jacket of PEBAX^{®} or the like is applied to the outside surface of the core by heat shrinking, coating, or the like. Typically, radiopaque markers are placed on the tip portion. At the proximal end, a luer locking hub is typically provided to enable connection of the guidewire support catheter to an infusion pump to deliver saline solution, an imaging contrast agent, or the like through the guidewire lumen of the guidewire support catheter. Saline solution, for example, advantageously provides lubrication to assist in running the guidewire support catheter up the guidewire.

The threading can be applied to the distalmost 3-6 mm or so of the guidewire support catheter in various ways. In one approach, an adhesive is used. In another approach, the radiopaque markers are in the form of swage rings that are swaged onto the tip and double as fasteners for holding the threading onto the tip. In a third disclosed approach, the outer jacket is applied over both the braided or helical stainless steel core and the threading, so as to hold the threading onto the tip.

In a further aspect, the extreme tip of the guidewire support catheter may be tapered to further assist in the crossing process.

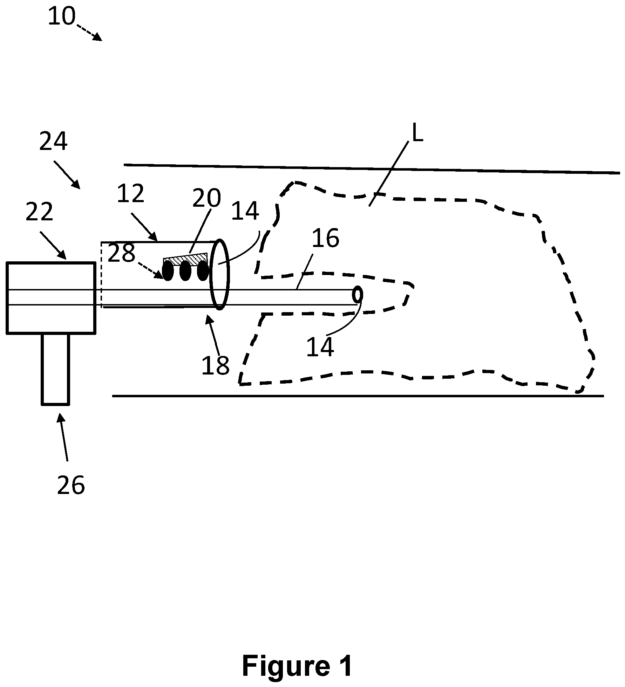

With reference to FIGURE 1, an illustrative intravascular lesion crossing device 10 insertable into a blood vessel V for treating a lesion L (or a clot, or an occlusion, and so forth) in the blood vessel V is diagrammatically shown. As shown in FIGURE 1, the device 10 includes a guidewire support catheter 12 having a guidewire lumen 14 configured to receive an associated guidewire 16 for delivery of a distal end 18 of the guidewire support catheter to an occlusion (i.e., the lesion L) in the blood vessel V. In some examples, the distal end 18 of the guidewire support catheter 12 has a tapered tip 19 to better navigate through the blood vessel V and to force its way through the lesion L. The guidewire lumen 14 can have an inner diameter sized to pass a guidewire of a chosen guidewire diameter, such as a guidewire 16 having a standard 0.236 mm, 0.457 mm, 0.889 mm (0.014", 0.018", or 0.035") guidewire diameter.

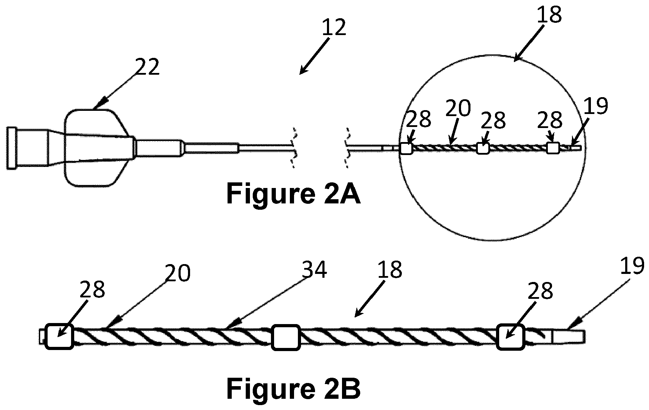

With continuing reference to FIGURE 1 and further reference to FIGURES 2A and 2B, the guidewire support catheter 12 is further illustrated and described. FIGURE 2A shows an isolation view of the guidewire support catheter 12, while FIGURE 2B shows an enlarged view of the distal end 18 of the guidewire support catheter 12 of FIGURE 2A. A helical profile 20, is provided at the distal portion of the guidewire support catheter 12 on an outer surface of the distal portion. The helical profile may be a helical threading 20 disposed at the distal portion of the guidewire support catheter. The helical threading 20 wraps around the outside of the distal portion of the guidewire support catheter 12. The helical threading 20 can have a length, for example, of 3-6 cm, although other values for the length greater or less than this illustrative range are contemplated, and can comprise metal or polymer or so forth. The guidewire support catheter 12 is rotatable about the associated guidewire 16 disposed in the guidewire lumen 14. Rotation of the distal end 18 of the guidewire support catheter 12 is operative to drive the distal end 18 into the lesion L in the blood vessel V by action of the helical threading 20. As best seen in the enlarged view of the distal end 18 of the guidewire support catheter 12, one or more (illustrative three) radiopaque markers 28 may be provided on the distal portion of the guidewire support catheter 12 to aid in its visibility in an interventional imaging modality used in the intravascular procedure employing the catheter 12. By way of nonlimiting illustrative example, the radiopaque markers 28 may be made of a material with high contrast in X-ray imaging, such as platinum, iridium, alloys thereof, and/or so forth.

As further shown in FIGURE 1 and FIGURE 2A, a luer locking hub 22 is disposed at a proximal end 24 of the guidewire support catheter 12, and is configured to connect the guidewire support catheter 12 to an associated infusion pump 26 to supply fluid for treating the lesion L.

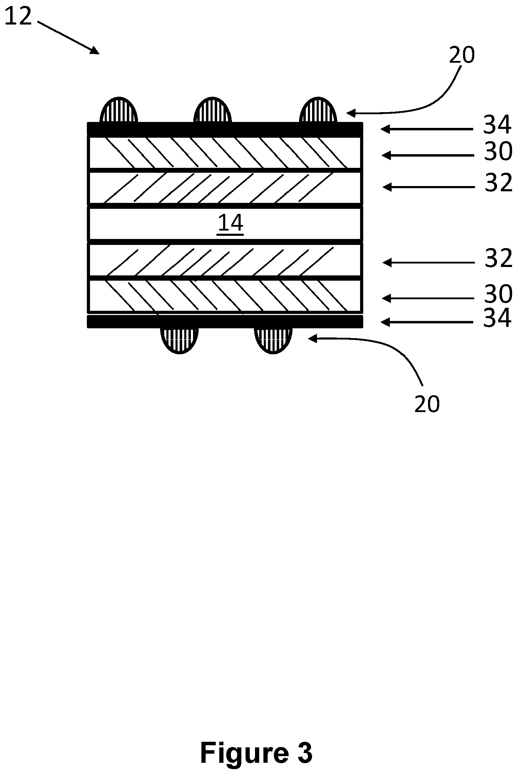

Referring now to FIGURE 3, and with continuing reference to FIGURES 1, 2A, and 2B, a longitudinal cross-sectional view, i.e., side sectional view. of a portion of the distal portion of a guidewire support catheter 12 is diagrammatically shown. The guidewire support catheter 12 comprises a cylindrical core 30 comprising a metal (i.e., stainless steel), and can comprise braids or helical strands. An inner jacket 32 is disposed on an inside surface of the cylindrical core 30, and comprises a polymeric material such as plastic (e.g., PTFE or Teflon^{™}). The inner jacket 32 provides a low-friction inside surface for the guidewire lumen 14, and thus reduces frictional resistance to movement of guidewire through the guidewire lumen 14. An outer jacket 34 surrounds the outside of the cylindrical core 30, and comprises a polymeric material such as plastic (e.g., PEBAX^{®}). The outer jacket 34 should be biocompatible, and provides a low friction surface with the lumen of the blood vessel to reduce friction as the guidewire support catheter 12 passes through the blood vessel during insertion of the catheter as part of the intravascular procedure. In the embodiment of FIGURE 3, the helical threading 20 is disposed on an outside of the outer jacket 34; however, as will be described, in other embodiments the helical threading could be inside the outer jacket.

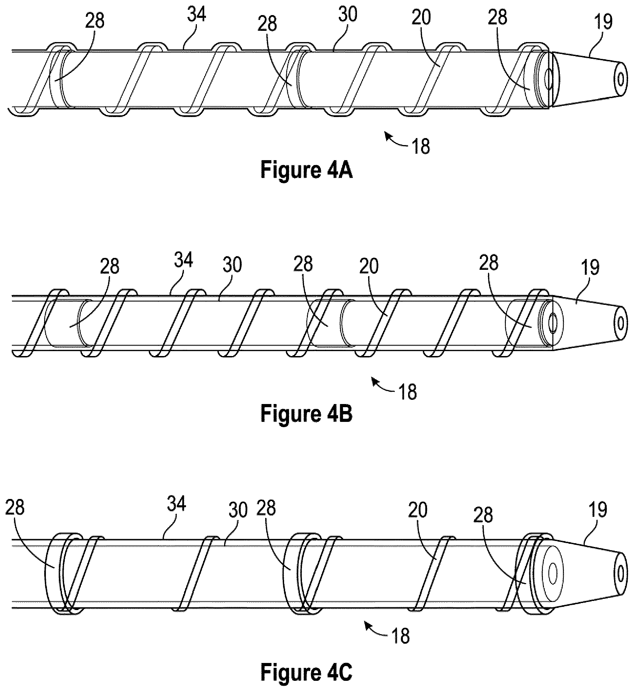

FIGURES 4A, 4B, and 4C diagrammatically illustrate three embodiments of the distal portion of a guidewire support catheter suitably used in the crossing device FIGURE 1. Each of these embodiments includes the cylindrical core 30 with tapered tip 19 surrounded by the outer jacket 34. The inner jacket 32 shown in FIGURE 3 is located inside the core 30 and hence is not visible. In FIGURES 4A, 4B, and 4C, the outer jacket 34 is shown as transparent for illustrative purposes.

FIGURE 4A shows an embodiment in which the helical threading 20 is wrapped around the cylindrical core 30 first, and then is covered by the outer jacket 34, which in this embodiment is a heat shrunk outer jacket 34. The heat shrunk outer jacket 34 thus secures the helical threading 20 to the distal portion of the catheter. In this embodiment, the radiopaque markers 28 are also installed prior to applying the shrunk outer jacket 34. An advantage of this embodiment is that the helical threading 20 and the radiopaque markers 28 are contained within the outer jacket 34, thus providing a low friction surface.

FIGURE 4B shows an embodiment in which the outer jacket 34 is applied to the cylindrical core 30 first, for example by a heat shrinking process. The helical threading 20 is then adhered to the outside surface of the outer jacket 34. The radiopaque markers 28 can be installed either before or after the outer jacket 34.

FIGURE 4C shows an embodiment in which the radiopaque markers 28 are in the form of marker bands 28 that are swaged onto the distal portion after the helical threading 20 is wrapped around the cylindrical core 30. The swaged marker bands 28 thus serve as fasteners to secure the helical threading 20 on the distal end 18 of the catheter. As shown in FIGURE 4C, the outer jacket 34 is installed before both the helical threading 20 and the swaged marker bands 28, although as the outer jacket is not serving as a fastener here it could be installed at a later time (e.g., after the helical threading 20 is applied).

FIGURES 4A, 4B, and 4C are merely illustrative examples, and other manufacturing processes are contemplated.

The helical threading 20 may not extend over the tapered tip 19 of the distal end 18 of the guidewire support catheter 12. It is also not necessary for the entire length of the guidewire support catheter 12 to be threaded. The helical threading 20 can span an entire length of the guidewire support catheter 12 between radiopaque markers 28 (e.g., 3 cm to 10 cm in some nonlimiting illustrative embodiments). This allows a medical professional to easily visualize the location of the threads with fluoroscopy and places the helical threading 20 close to any lesion or occlusion the medical professional is working to cross.

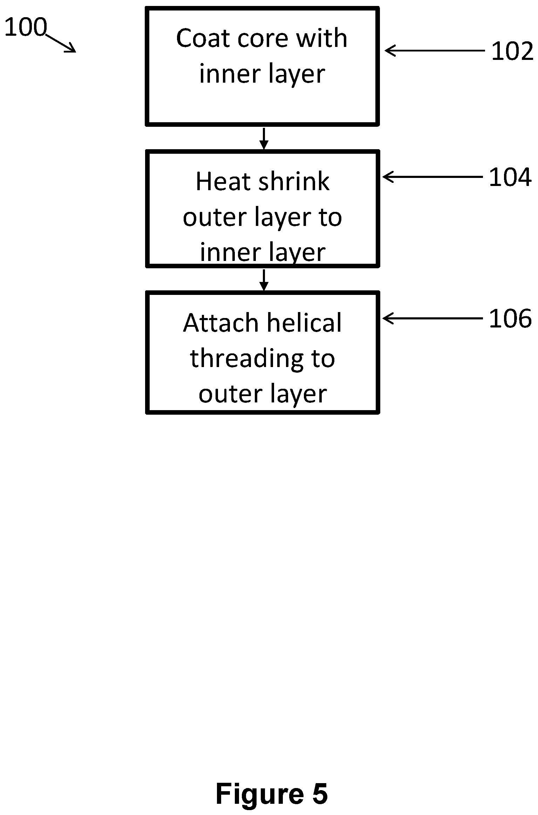

Referring to FIGURE 5, an illustrative embodiment of a method or process 100 of forming the intravascular lesion crossing device 10 is diagrammatically shown as a flowchart. At an operation 102, an inner layer (i.e., the inner jacket 32) is coated on the inside surface of the cylindrical core 30. At an operation 104, an outer layer (i.e.., the outer jacket 34) is heat-shrunk to the outer surface of the cylindrical core 30. At an operation 106, the helical threading 20 is attached to the outer jacket 34 (corresponding to the embodiment of FIGURE 4B), or is wrapped around the outer jacket and then secured by swaging the radiopaque markers 28 (embodiment of FIGURE 4C). For the embodiment of FIGURE 4A, the order of operations 104 and 106 shown in FIGURE 5 is reversed, i.e. the helical threading 20 is first attached to the core 30 (corresponding to operation 106) followed by the outer jacket 34 being heat-shrunk to the inner jacket 32 and thereby securing the helical threading 20.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. An intravascular lesion crossing device (10), comprising:
a guidewire support catheter (12) having a guidewire lumen (14) configured to receive an associated guidewire (16) for delivery of a distal end (18) of the guidewire support catheter to an occlusion in a blood vessel; and
a helical profile (20) provided at an outer surface of a distal portion of the guidewire support catheter.

2. The intravascular lesion crossing device (10) of claim 1, wherein the helical profile (20) of the guidewire support catheter (12) has a length of 3-6 cm.

3. The intravascular lesion crossing device (10) of claim 1 or 2, wherein the guidewire support catheter (12) comprises:
a cylindrical core (30);
an inner jacket (32) disposed on an inside surface of the cylindrical core; and
an outer jacket (34) surrounding the cylindrical core;
wherein the helical profile is provided as helical threading (20) disposed on an outside of the outer jacket.

4. The intravascular lesion crossing device (10) of claim 1 or 2, wherein the guidewire support catheter (12) comprises:
a cylindrical core (30);
an inner jacket (32) disposed on an inside surface of the cylindrical core; and
an outer jacket (34) surrounding the cylindrical core;
wherein the helical profile is provided as helical threading (20) disposed between the cylindrical core and the outer jacket.

5. The intravascular lesion crossing device (10) of claim 3 or 4, wherein the cylindrical core (30) comprises a metal.

6. The intravascular lesion crossing device (10) of any one of claims 3-5, wherein the cylindrical core (30) comprises braids or helical strands.

7. The intravascular lesion crossing device (10) of any one of claims 3-6, wherein the inner jacket (32) and the outer jacket (34) each comprise a polymeric material, preferably a plastic material.

8. The intravascular lesion crossing device (10) of any one of claims 3-7, further comprising:
one or more radiopaque markers (28) disposed on the distal portion of the guidewire support catheter (12).

9. The intravascular lesion crossing device (10) of claim 8, wherein the radiopaque markers fasten the helical threading (20) on the distal portion of the guidewire support catheter.

10. The intravascular lesion crossing device (10) of any one of claims 3-9, wherein the helical threading (20) is secured either to the outside of the outer jacket (34) or to the cylindrical core by an adhesive.

11. The intravascular lesion crossing device (10) of any one of claims 3-10, further comprising:
a luer locking hub (22) disposed at a proximal end (24) of the guidewire support catheter (12) and configured to connect the guidewire support catheter to an associated infusion pump (26).

12. The intravascular lesion crossing device (10) of any one of claims 1-11, wherein the distal end (18) of the guidewire support catheter (12) has a tapered tip.

13. The intravascular lesion crossing device (10) of any one of claims 1-12, wherein the helical threading (20) comprises metal or polymer.

14. The intravascular lesion crossing device (10) of any one of claims 1-13, further comprising the guidewire (16), and wherein the guidewire support catheter (12) is configured rotatable about the associated guidewire (16) disposed in the guidewire lumen (14) of the guidewire support catheter, whereby rotation of the distal portion of the guidewire support catheter is operative to drive its distal end into the occlusion in the blood vessel by action of the helical profile.

15. A method (100) of forming a catheter (12), the method comprising:
coating a core layer (30) with an inner layer (32);
providing an outer layer (34) onto the core layer; and
securing a helical threading (20) to the outer layer or
securing the helical threading to the core layer.
